# EUROPEAN PATENT APPLICATION

(11) **EP 3 000 450 A1**
(43) Date of publication of application: **30.03.2016**
(21) Application number: 14075062.1
(22) Date of filing: 23.09.2014
(51) Int. Cl.: A61F 13/49, A41B 9/12, A61F 13/66, A61F 13/15

(54) **Protective and reusable underwear for retention of liquid discharge related to haemorrhoids**

(71) Applicant: Sprachmann, Gerald, 1019 DV Amsterdam (NL)
(72) Inventor: Sprachmann, Gerald, 1019 DV Amsterdam (NL)

(57) **Abstract**

The present invention relates to underwear to be worn by a user for absorbing liquid rectal discharge related to hemorrhoids or any other rectal disease. In particular, the present invention relates to a novel underwear design that incorporates a non removable, washable composite material that absorbs the uncontrolled discharged liquid and prevents liquid from passing through the underwear.

The washable composite material consists of two main layers. One, that faces the outside of the underwear, is a waterproof but breathable layer to prevent liquid material to pass through the underwear. The other one, that faces the inside of the underwear, is an absorption material that takes up the discharged liquid. Due to the breathable nature of the waterproof layer, water vapor can pass through it and allows therefore the absorption layer to dry off. This provides a comfortable dry feeling on the skin and additionally protects the user from uncontrolled liquid release through the underwear.

The composite material, compromising the absorbent material and breathable water proof material, has a sufficient absorption capacity for a typical daily liquid discharge related to hemorrhoids or rectal diseases. The underwear, together with the incorporated composite material can undergo multiple washing and drying cycles. All materials of construction allow high washing temperatures (60-95 degree Celsius) for disinfection and to destroy unpleasant odor. The composite material is incorporated in the underwear around the anal area and the material is thin and soft making if comfortable to wear. Additional the composite material is incorporated such in the underwear that it is not visible from the outside or inside of the underwear. This provides the user with the additional benefit of intimacy.

The present invention will therefore simplify the life of numerous people and give them protection against uncontrolled rectal liquid discharge.

## Description

### Background of the Invention

Up to half of the men population may experience problems with hemorrhoids at some point in their lives. The unpleasant consequence of haemorrhoids is the uncontrolled liquid discharge from the anus. Such liquid discharge can contain blood, mucous, water, stool, purulent and any combinations of these substances. The liquid discharge related to hemorrhoids can happen instantly without allowing the individual person to take protective measures in time. Such a protective measure can be the use of a dischargeable adsorbent pad. The advantage of the present invention is that it provides protection when the instant rectal liquid discharge occurs without having a negative effect on comfort. The present invention can therefore be used on a daily basis without the need of taking measures in the event of an uncontrolled liquid discharge.

### Field of the Invention

The present invention relates to clothing underwear and more specifically to reusable underwear with an incorporated, washable composite material (absorption layer and breathable waterproof layer) to control rectal liquid discharge related to hemorrhoids or any other rectal disease. There are various devices in this field dealing with the control of body fluids. However none of them consider an incorporated and washable composite material that is specially designed to withhold rectal liquid discharge. The present invention is the first one where the absorbent material doesn't need to be removed. The underwear can be washed and reused together with the composite material (absorption material and breathable waterproof material) and can undergo multiple washing and drying cycles at temperatures between 60 and 95 degree Celsius. This allows an effective destruction of bacteria and odor. An additional benefit of the present invention is the breathable nature of the waterproof layer where liquid material cannot pass through but water vapor can. This allows the absorption layer to dry off and additionally provides the user with a dry and comfortable feeling on the skin.

### Description of Prior Art

U.S. Patent No. 6,393,621 to Redwine, et al . This patent discloses underwear useful with an absorbent article, particularly a menstrual pad of the type that has an adhesive layer covered with a peel off strip that is removed so that the pad can be adhered to the crotch area of the underwear.

U.S. Patent No. 3,489,149 to Larson , which discloses a menstrual panty having a pocket sewn into the crotch area, the pocket being for receiving an absorbent disposable menstrual pad

U.S. Patent No. 4,352,356 to Tong discloses a urinary incontinence panty having a pouch connected in the crotch area and adapted to receive an absorbent disposable urinary incontinence pad.

U.S. Patent No. 3,613,686 to Woskin discloses a sanitary panty garment with a pocket in the crotch area.

### Summary of the Invention

The disclosed invention considers protective, reusable underwear for the absorption of liquid rectal discharge products. The underwear contains a washable, non removable composite material (absorption layer and breathable waterproof layer) that is incorporated around the anal area. The absorption layer is made of a soft material such as cotton, flannel, silk, fleece, polyester or any other liquid retaining material. The breathable waterproof layer is used to prevent liquid discharge material from passing through the underwear to the outside. Examples for washable and breathable waterproof materials are Teflon, nylon, polyurethane or any other breathable waterproof material. The breathable waterproof material itself can consist of several layers. Additional the breathable waterproof material can contain a membrane layer. The breathable waterproof material faces the outside of the underwear and the liquid absorption material faces the inside of the underwear. Due to the breathable nature of the waterproof layer, water vapor can pass through it and allows therefore the liquid absorption layer to dry off. This provides a comfortable dry feeling on the skin and additionally protects the user from uncontrolled liquid release through the underwear.

The underwear, comprising composite material can undergo multiple washing cycles at temperatures between 60 and 95 degree Celsius to destroy bacteria and unpleasant odor.

The primary objective of the invention is to provide protective underwear against uncontrolled liquid discharge related to hemorrhoids or any other anal disease. The invention overcomes the problem of instant discharge of fluids as the underwear can be used on a daily basis without losing comfort. An additional objective of the invention is that the composite material is washable and therefore reusable. Another objective of the invention is that the composite material is breathable to allow water vapor to pass through. Another objective of the invention is that the composite material is not visible from the outside or inside of the underwear. Yet another objective of the underwear is to have sufficient absorption capacity for a typical daily liquid discharge related to hemorrhoids or rectal diseases.

### Brief Description of Drawings

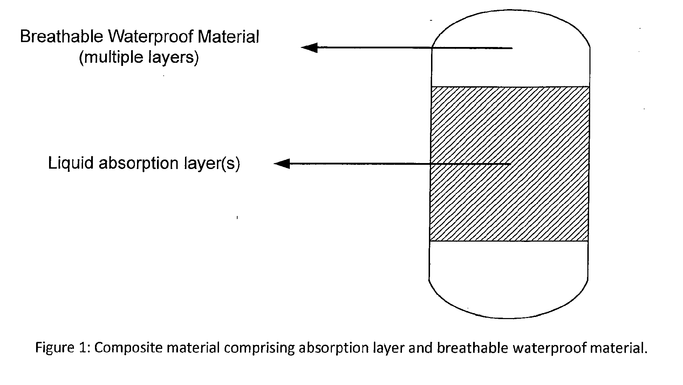

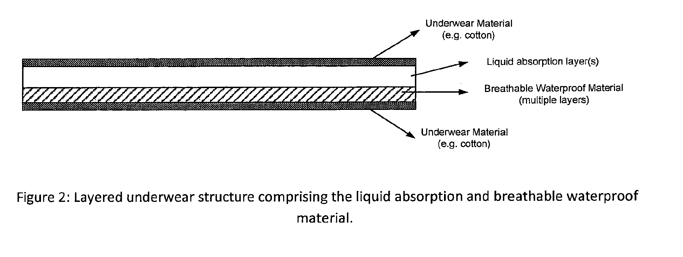

Figure 1 and Figure 2 show the layered structure of the incorporated composite material (absorption material and breathable waterproof material). The breathable waterproof material faces the outside of the underwear whereas the absorption material faces the insides (anus) of the underwear. Both materials can contain one or more layers of different materials. The composite material is further incorporated in the underwear and covered by the underwear material (shown in Figure 2). It is therefore not visible from the inside or outside of the underwear.

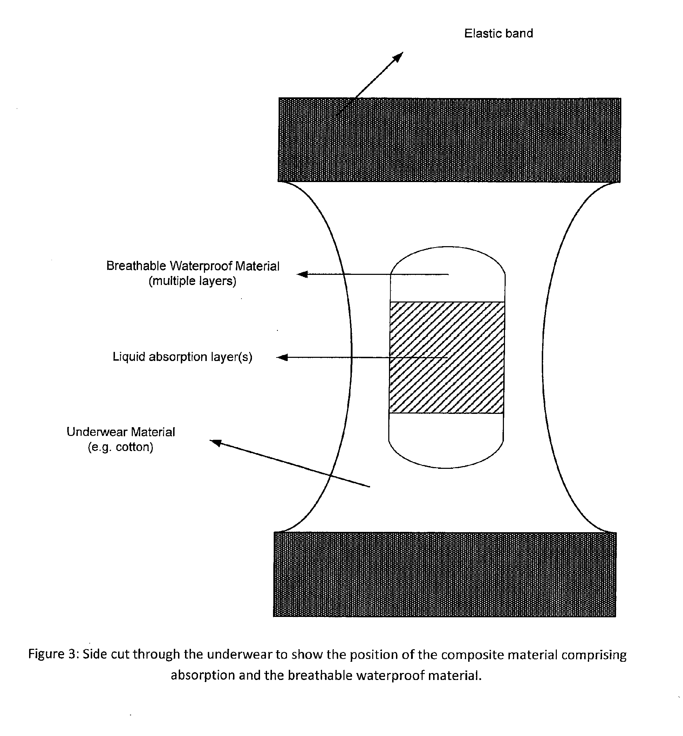

Figure 3 shows in a cut view the position of the composite material (absorption material and breathable waterproof material) in the underwear. The composite material can cover a narrow area around the anus but it can also cover wide area depending on the specific design and application.

## Claims

1. A washable, reusable and protective underwear for the retention of liquid discharge related to hemorrhoids or any other rectal disease comprising of:
a. An incorporated, washable absorption material for the retention of liquid rectal discharge
b. An incorporated washable and breathable waterproof material to prevent liquid discharge from permeating through the underwear.
c. An incorporated washable and breathable waterproof material that allows water vapor to pass through.
d. A washable, reusable underwear where the adsorption material and the breathable waterproof material are incorporated.

2. An absorption material that is made of cotton, fleece, silk, polyester or any other liquid absorbing material.

3. An absorption material that consist of several layers of material.

4. A breathable waterproof material that is made of Teflon, nylon, polyurethane or any other non liquid permeating material.

5. A breathable waterproof material that consist of several layers of material.

6. A breathable waterproof material that contains a membrane.

7. Underwear incorporating the composite material (absorption material and breathable waterproof material) that can undergo multiple washing cycles at temperatures between 30 and 95 degree Celsius preferably between 50 and 95 degree Celsius most preferably between 60 and 95 degree Celsius.

8. An underwear that provides sufficient absorption capacity for a typical daily liquid discharge related to hemorrhoids or rectal diseases.
